# FASCICULE DE BREVET EUROPEEN

(11) **EP 2 502 607 B1**
(45) Date de publication et mention de la délivrance du brevet: **22.03.2017**
(21) Numéro de dépôt: 12160773.3
(22) Date de dépôt: 22.03.2012
(51) Int. Cl.: A61F 2/64, A61F 5/01, F16D 41/08, A61F 2/68, A61F 2/50

(54) **Dispositif de contrôle de l'articulation d'une prothèse de membre inférieur**
Kontrollvorrichtung des Gelenks einer Prothese der unteren Gliedmaßen
Device for controlling the joint of a lower limb prosthesis

(30) Priorité: 22.03.2011 FR 1152351
(43) Date de publication de la demande: 26.09.2012
(73) Titulaire: Henri Blanc, 33850 Leognan (FR)
(72) Inventeur: Blanc, Henri, 33140 Mérignac (FR); Ochsenhofer, Alain, 33140 Villenave d'Ornon (FR); Klotz, Rémi, 33320 Eysines (FR)
(74) Mandataire: Aquinov

(56) Documents cités:
- GB-A- 344 430
- GB-A- 2 161 386
- US-A- 2 594 227
- US-A- 4 451 939
- US-A1- 2006 211 966

## Description

La présente invention concerne les appareils prothétiques et plus particulièrement les prothèses articulées de remplacement d'un membre.

### ARRIERE PLAN DE L'INVENTION

Dans le domaine de l'orthopédie, on a recours à des prothèses de membres, typiquement une jambe articulée pour un sujet amputé fémoral, ou des orthèses qui viennent en assistance au niveau de l'articulation d'un membre affecté.

L'articulation d'un membre artificiel doit être contrôlée pour qu'elle puisse se comporter de manière satisfaisante lors du déplacement du sujet prothésé. Ainsi par exemple, pour un sujet amputé du fémur, il convient d'empêcher le repliement de la jambe sous ou en direction de la cuisse, lorsque la prothèse prend en charge tout ou partie du poids du sujet et ce quelle que soit la valeur de l'angulation de la prothèse à l'instant où commence ce transfert de poids.

Par ailleurs, il peut être utile de pouvoir immobiliser un genou naturel opéré ou traumatisé au moyen d'une orthèse dont on règle et contrôle l'angulation en fonction de l'évolution du genou dans le temps.

Il existe des dispositifs qui assurent le blocage ou le freinage d'une articulation pour maintenir l'angulation des deux éléments articulés alors que ceux-ci sont soumis à un couple autour de l'articulation. Certains d'entre eux mettent en oeuvre des mécanismes de verrouillage externes à l'articulation, notamment des vérins hydrauliques de blocage, par fermeture de vannes appropriées en réponse à des capteurs sensibles aux efforts transmis à la prothèse , ou encore une transmission mécanique du blocage au moyen d'un câble ou d'une tige actionnés lors du contact de la prothèse avec le sol. De tels dispositifs sont par exemple divulgués dans US 2594227, US 2006/0211966, ou US 4451939.

L'invention s'inscrit dans une technologie plus simple et surtout plus compacte qui permet de diminuer sensiblement le volume et le poids de la prothèse .

### OBJET DE L'INVENTION

C'est ainsi que l'invention a pour objet un mécanisme d'articulation d'une prothèse de jambe comprenant :
- un élément destiné à être solidaire de la cuisse d'un sujet ;
- un autre élément destiné à être solidaire de la jambe de la prothèse , cet autre élément comprenant un corps et une palette basculante articulée sur ce corps au niveau d'un axe déporté vers l'avant selon le sens de la marche, cet autre élément étant destiné à être solidarisé à la jambe par la palette basculante ;
- une bague intérieure portée par l'un des éléments et une bague extérieure portée par l'autre élément ;
- les bagues étant accouplées à rotation par l'intermédiaire d'un embrayage à coincement intercalé entre elles et pourvu d'un moyen de neutralisation de l'accouplement des deux bagues comportant une roue de commande ;
- cette roue de commande étant accouplée par enroulement sans glissement, à au moins un lien d'indexation de la roue dans l'une ou l'autre de deux positions de celle-ci par rapport à l'embrayage ;
- une extrémité de ce lien étant fixée à l'élément solidarisé à la jambe par une fixation élastique agissant sur ce lien dans le sens de déblocage de l'embrayage ;
- l'autre extrémité de ce lien étant fixée à la palette basculante par l'intermédiaire d'un galet de renvoi porté par le corps, de sorte que la fixation élastique du lien tend à écarter la palette du corps et que le rabattement de la palette contre le corps agit sur le lien à l'encontre de son rappel élastique pour bloquer l'embrayage ;
- de telle manière qu'un appui sur le sol au niveau du talon du pied prolongeant la jambe de la prothèse tend à rabattre la palette pour bloquer l'articulation, et qu'un appui sur le sol au niveau de la pointe du pied tend à écarter la palette pour débloquer l'articulation.

Avantageusement, l'invention comporte en outre des moyens pour ajuster la position de la jambe de la prothèse sur la palette à laquelle cette jambe est fixée, ce qui permet d'ajuster la phase à laquelle l'articulation se débloque durant la marche, ainsi qu'un ressort de compression interposé entre la palette et le corps pour tendre continuellement à écarter la palette du corps.

Un embrayage à coincement est bien connu par exemple sous le nom de roue libre à corps de blocage.

De tels dispositifs sont par exemple divulgués dans US 2594227, US2006/0211966, US 4451939 ou GB 344430.

Dans la présente application le corps de blocage est formé par la bague intérieure qui comporte au moins une surface périphérique en regard de la surface intérieure de la bague extérieure formant un coin dans lequel, sous l'effet d'un ressort, un rouleau ou une bille vient se loger et interdit toute rotation relative des bagues dans le sens de la poussée du ressort. En revanche, une rotation relative dans l'autre sens tend à extraire le rouleau du coin dans lequel le pousse le ressort, ce qui permet la poursuite de cette rotation.

L'invention comporte en outre un moyen de neutralisation de l'accouplement en rotation des deux bagues qui empêche la roue libre de mettre en prise les deux bagues. C'est ce moyen qui comporte au moins la roue susdite, laquelle porte, pour chaque rouleau, entre les deux bagues, un doigt de limitation du déplacement des rouleaux sous l'effet des ressorts, mobile entre les deux positions susdites, c'est-à-dire entre une position qui est éloignée du coin susdit et une position qui est dans ledit coin pour que le rouleau puisse s'y loger.

La manoeuvre en rotation de la roue est donc assurée par le lien susdit qui, dans un mode de réalisation est formé par un câble qui s'enroule sans glissement sur une partie au moins de la périphérie de la roue, par exemple dans une gorge de celle-ci qui lui donne une fonction de poulie.

D'une manière générale, la palette basculante tend le lien à l'encontre de la fixation élastique lorsque cette palette est rabattue contre le corps qui la porte, ce qui a pour effet d'accoupler les deux bagues. Et cette palette libère le lien lorsqu'elle est écartée du corps, ce qui a pour effet de désaccoupler les deux bagues, car ce lien est alors assujetti à son moyen de fixation élastique.

En pratique, lorsque le patient appuie avec son talon sur le sol, il rabat la palette contre le corps qui la porte, ce qui provoque l'accouplement des deux bagues pour maintenir l'angle que forment alors le fémur et la jambe.

A la fin du pas, le patient appuie avec la pointe de son pied sur le sol, ce qui écarte la palette du corps qui la porte, provoquant le désaccouplement des deux bagues, ce qui permet au patient d'avancer sa cuisse, provoquant naturellement le repli de la jambe sous cette cuisse par effet d'inertie, jusqu'à placer son pied talon au sol pour le prochain pas.

Lorsqu'il est présent, le ressort de compression interposé entre la palette et le corps contribue aussi à assurer ce désaccouplement qui a également lieu aussi lorsqu'aucun contact n'existe entre le sol et le pied.

Dans un mouvement analogue, l'articulation selon l'invention permet au patient de monter des marches d'escalier de façon sensiblement naturelle.

L'invention permet ainsi de réaliser une jambe artificielle dont l'articulation se bloque automatiquement dans un sens qui sera le sens de la refermeture. On comprend en effet qu'il y aurait un grave inconvénient à ce que le repliement de la jambe se produise et qu'elle se dérobe sous le poids du sujet. Cet automatisme est assuré de manière simple et au moyen d'un mécanisme peu volumineux et donc de faible poids, ce qui est avantageux pour une telle prothèse.

Dans une autre variante de réalisation, adaptée à une orthèse, qui n'est pas l'objet de la présente invention telle que définie dans les revendications annexées, l'embrayage par coincement est double. Il s'agit d'un dispositif connu sous le nom de roue libre irréversible ou bidirectionnelle dans lequel l'espace entre les bagues extérieure et intérieure définit au moins une paire de coins opposés dans lesquels deux rouleaux sont normalement repoussés par un ressort. L'accouplement des deux bagues est ainsi bidirectionnel, donc dans les deux sens de rotation. Pour qu'un effet de roue libre soit créé, il convient de neutraliser l'action du ressort sur l'un des rouleaux de manière qu'il soit empêché de se loger dans le coin et autoriser une rotation relative des deux bagues qu'aurait interdite ce rouleau.

Un tel dispositif permet donc de maintenir une angulation entre deux organes articulés et de modifier cette angulation par pivotement dans le sens soit de l'ouverture de l'angle soit de sa fermeture, selon le rouleau que l'on retient hors du coin dans lequel il est normalement repoussé. Les moyens permettant ces découplages sélectifs comprennent deux jeux de doigts de limitation du déplacement des rouleaux, portés par au moins une roue actionnée par un câble, une traction d'une extrémité de ce câble libérant un sens de rotation de l'articulation. Deux roues peuvent encadrer les bagues et un câble est attaché à chaque roue par une extrémité, l'autre extrémité étant libre pour la commande de déblocage par un opérateur.

L'intérêt de ce dispositif réside dans le caractère continu du réglage possible alors que les dispositifs connus mettent en oeuvre des crantages qui ne permettent que des angulations progressant de manière discrète.

D'autres caractéristiques et avantages de l'invention ressortiront de la description donnée ci-après d'un exemple de réalisation de l'invention.

### BREVE DESCRIPTION DES DESSINS

Il sera fait référence aux dessins annexés dans lesquels :
- la figure 1 est un schéma fonctionnel en coupe antéro-postérieure d'une première réalisation du mécanisme de l'invention mis en oeuvre dans une jambe artificielle ;
- la figure 2 est un schéma orthogonal au premier de ce premier mécanisme ;
- la figure 3 est un schéma fonctionnel en coupe également antéro-postérieure d'une deuxième réalisation non revendiquée, adaptée à une orthèse ;
- la figure 4 est un schéma orthogonal de cette deuxième réalisation ;
- la figure 5 est une vue latérale montrant l'articulation selon l'invention avant le début d'un nouveau pas lorsque le porteur de cette articulation s'apprête à poser son talon au sol provoquant par là même le blocage de l'articulation ;
- la figure 6 est une vue latérale montrant l'articulation selon l'invention à la fin d'un pas lorsque le porteur de cette articulation appuie sur le sol avec la partie avant de son pied dans une phase qui précède le déblocage de l'articulation.

### DESCRIPTION DETAILLEE DE L'INVENTION

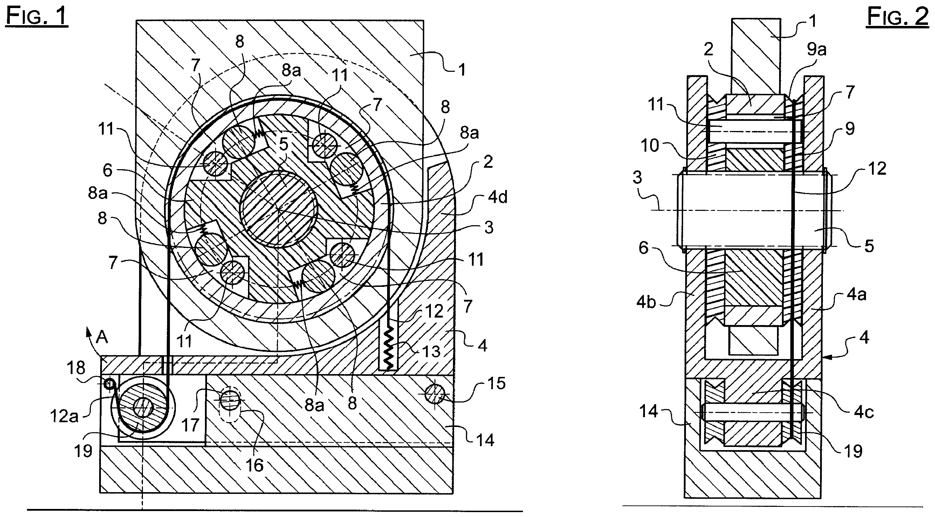

La pièce 1 représentée aux figures 1 et 2 représente la partie du mécanisme d'articulation qui est reliée à la cuisse du sujet. Les moyens de liaison ne sont pas représentés. Cette pièce est solidaire d'une bague 2 dite bague extérieure dont l'axe 3 est l'axe de pivotement de l'articulation, donc du genou dans une jambe artificielle.

La pièce 4 constitue le corps de la pièce du mécanisme de l'invention qui est solidaire de la jambe de la prothèse. Elle est en forme de chape avec deux ailes 4a et 4b entre lesquelles est logée la bague 2. Cette chape possède une base 4c qui relie les deux ailes et qui se prolonge à l'avant de l'articulation par une extension 4d qui forme butée antérieure pour la rotation relative des pièces 1 et 4 limitant ainsi l'extension de la jambe.

La pièce 4 porte un arbre 5, coaxial à l'axe 3, sur lequel est montée une bague intérieure 6 logée entre les deux ailes et dont la périphérie est centrée dans la bague extérieure 2. La largeur des bagues est sensiblement identique et inférieure à la distance séparant les ailes 4a et 4b de la pièce 4. La bague intérieure 6 et la pièce 4 sont calées en rotation l'une par rapport à l'autre, l'arbre 5 étant par exemple cannelé à cet effet. Il faut noter que la surface périphérique de la bague intérieure 6 est pourvue d'encoches 7 dans lesquelles sont logés des rouleaux 8 pour former de manière connue une liaison à roue libre entre les bagues 2 et 6. Pour ce faire, chaque encoche définit une surface de fond d'encoche qui converge vers la surface intérieure de la bague extérieure de sorte que le rouleau correspondant vient se coincer sous l'effet d'un ressort 8a, entre les deux bagues et les immobiliser l'une par rapport à l'autre si leur mouvement relatif tend à coincer ce rouleau entre le fond d'encoche et la bague extérieure, en agissant sur le rouleau dans le même sens que le ressort. Dans le cas de la figure 1, il s'agit du mouvement de repliement de la jambe sous la cuisse (flèche A). Un pivotement relatif inverse est libre.

Dans la configuration représentée à la figure 1, la pièce 1 est en butée contre l'extension antérieure 4d de la pièce 4 et est bloquée par le coincement des rouleaux 8 dans l'encoche 7. Or il convient que cette articulation soit libre tant que la prothèse n'est pas soumise au poids du sujet. Ce dernier doit pouvoir, lors de la marche, lancer sa jambe vers l'avant laquelle doit pouvoir se placer en extension. Lors d'une montée d'escalier, la jambe doit pouvoir naturellement, sous l'effet de la gravité, pendre à l'extrémité de la cuisse qui est relevée autour de la hanche. En d'autres termes, il faut que l'articulation soit libre si la jambe est « en l'air ».

Ce résultat est obtenu dans l'invention par un mécanisme d'inhibition de l'effet roue libre. Il s'agit d'au moins un roue et de préférence comme représenté, de deux roues 9 et 10 qui encadrent les bagues 2 et 6 entre les ailes 4a et 4b de la pièce 4 et qui sont montés tournants sur l'arbre 5. Ces roues sont porteuses de doigts 11, un par encoche, qui s'étendent donc dans ces encoches 7 qui comportent à cet effet un espace au-delà du coin qu'elles forment et à l'opposé des ressorts 8a. Un pivotement de ces roues autour de l'arbre 5 permet de pousser les doigts 11 contre les rouleaux 8 en écrasant les ressorts 8a et ainsi, en les dégageant de leur coincement. Le pivotement relatif des pièces 1 et 4 est alors entièrement libre dans les deux sens. Le pivotement de ces roues est commandé par un câble 12 (ou deux câbles jumelés, un par roue) qui s'enroule sans glisser dans une gorge périphérique 9a du roue 9, cette dernière jouant alors le rôle d'une poulie d'entraînement des doigts 11.

En l'absence de charge sur le talon de la jambe, le câble 12 est tiré par un organe élastique 13 qui attache une extrémité du câble à la pièce 4 et qui force les doigts 11 à extraire les rouleaux 8 de leur position de coincement. L'autre extrémité du câble 12 est destinée à recevoir une force de traction qui s'oppose à l'effet de l'organe 13 de manière à faire pivoter la roue 9 pour écarter les doigts 11 des rouleaux 8 qui se coincent alors sous l'effet des ressorts 8a entre les deux bagues en interdisant le repliement de la jambe sous la cuisse. Cette force de traction résulte typiquement du contact du talon de la prothèse avec le sol.

Les figures 1 et 2 montrent également une palette 14 articulée à la pièce 4 autour d'un axe 15 antérieur perpendiculaire au plan antéro-postérieur de la prothèse donc parallèle à l'axe 3 en étant situé en avant de celui-ci par rapport au sens de la marche. Cette palette qui constitue une portion mobile de la pièce solidaire de la jambe de la prothèse, peut donc ainsi pivoter par rapport à la pièce 4 autour de l'axe 15 mais sur une faible amplitude qui est définie par une lumière 16 de la pièce 4 dans laquelle peut se déplacer un doigt 17 solidaire de la palette.

Dans sa partie postérieure, la palette 14 porte un organe d'attache 18 de l'extrémité libre 12a du câble 12. Cette extrémité passe sur un galet 19 de renvoi qui est porté par la pièce 4.

Avantageusement, le système comporte encore un ressort de compression, interposé entre la palette 14 et le corps 4 de manière à tendre continuellement à écarter la palette du corps 4. Ce ressort tend continuellement à débloquer l'articulation, et il contribue en outre à adoucir le choc résultant du talon entrant en contact avec le sol durant la marche.

A la figure 1, le mécanisme est représenté bloqué, c'est-à-dire que le pied de la jambe repose par son talon sur le sol. Le blocage résulte du rabattement de la palette 14 contre la portion 4c de la pièce 4. Ce rabattement entraîne une traction sur le câble 12 puisque l'attache 18 remonte derrière le galet 19. Les doigts 11 ont donc libéré les rouleaux 8 qui viennent se coincer entre les deux bagues 2 et 6. La jambe en charge ne peut donc pas se replier intempestivement.

Dès que la jambe quitte le sol, l'organe élastique 13 ainsi que le ressort de compression rappellent le câble 12 et forcent le doigt 17 contre l'extrémité supérieure de la lumière 16. La palette est alors légèrement basculée sous la pièce 4 et, dans le même temps, les roues 9 et 10 sont pivotées par rapport à la bague 6 et les doigts 11 éloignent les rouleaux 8 de leur position de coincement. La jambe est lors libre de pivoter dans les deux sens par rapport à la cuisse. La hauteur du point 18 d'attache de l'extrémité du câble 12 peut avantageusement être prévue ajustable pour affiner le réglage du déclenchement du blocage et du déblocage de l'articulation durant la marche.

Un nouveau contact au sol du talon du pied de la prothèse rabattra la palette 14 contre la pièce 4 et mettra immédiatement l'articulation en position d'interdiction de la poursuite d'un repliement.

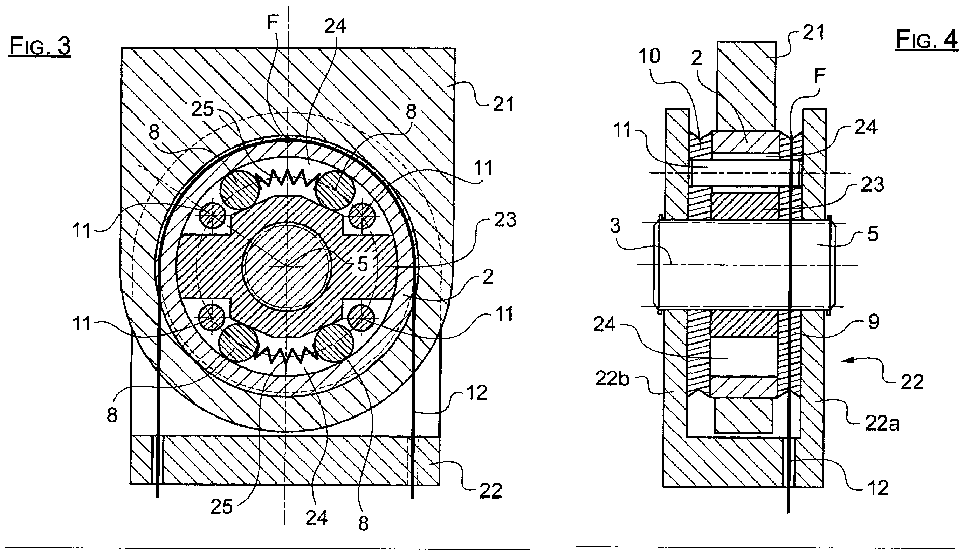

Aux figures 3 et 4, on retrouve certains des éléments déjà décrits avec les mêmes références. Il s'agit dans cette réalisation, non couverte par les revendications annexées, d'une articulation entre deux éléments d'orthèse, articulation à mécanisme de blocage dans n'importe quelle angulation des éléments.

La pièce 21 est liée à la cuisse. Elle possède la bague extérieure 2. La pièce 22 est liée à la jambe. Elle possède la bague intérieure 23 liée aux ailes 22a et 22b par l'arbre cannelé 5. Cette bague intérieure 23 coopère avec la bague 2 au moyen de rouleaux de coincement 8 et d'encoches 24 qui diffèrent de celles précédemment décrites au sens où deux rouleaux 8 consécutifs viennent se loger dans des espaces de coincement opposés sous l'effet de ressorts 25. Les roues 9 et 10 portent les doigts 11 qui, à partir d'une position médiane où ils ne coopèrent pas avec les rouleaux et pour laquelle l'articulation est bloquée, ils décoincent selon le sens de rotation des roues, les rouleaux 8 qui libèrent uniquement un sens de pivotement des pièces 21 et 22. Cet agencement est connu sous le nom de roue libre bidirectionnelle ou irréversible.

Ici le câble 12 permet l'actionnement des roues dans les deux sens en coopérant sans glisser (point de fixation F par exemple) dans la gorge périphérique de l'un d'eux. Ainsi, quand un opérateur tire sur une extrémité extérieure au mécanisme de l'un des câbles l'orthèse ne peut qu'être ouverte alors que la manoeuvre de l'autre extrémité de ce câble ne permet que la fermeture de l'articulation. On peut prévoir deux câbles, un par roue, l'un affecté à la manoeuvre des doigts dans un sens et l'autre pour leur manoeuvre dans l'autre sens, chacun d'eux étant fixé à la roue correspondante.

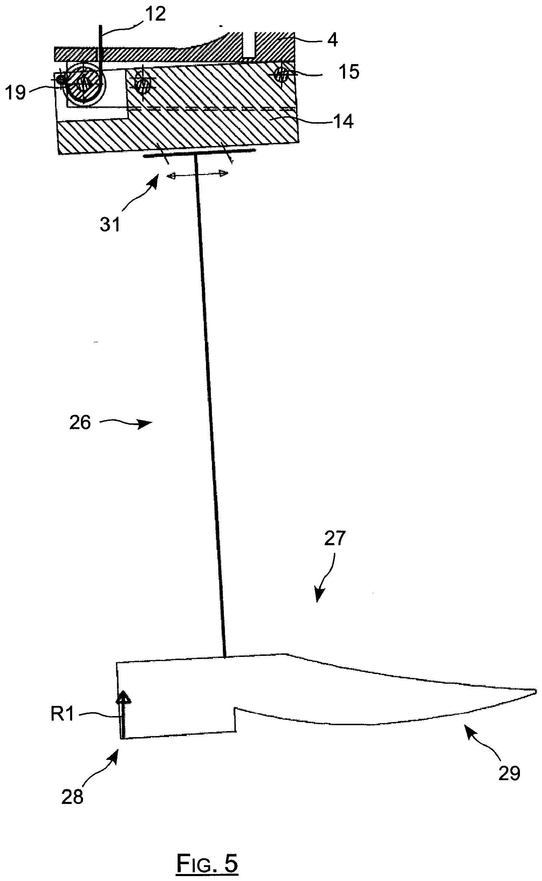

Comme illustré à la figure 5, la jambe 26 est rigidement solidarisée à la palette 14, de sorte qu'elle est sensiblement en arrière par rapport à l'axe 15 par lequel cette palette 14 est articulée sur le corps 4.

Lorsque le porteur de cette articulation appuie sur le sol par la partie arrière 27 de son pied 28, il tend ainsi à rabattre la palette 14 contre le corps 4, et lorsqu'au contraire il appuie sur le sol avec la partie avant 29 de son pied 28, l'axe d'application des efforts est déporté vers l'avant par rapport à l'axe 15, ce qui tend au contraire à écarter la palette 14 du corps 4.

Ainsi, durant la marche, lorsque le porteur de l'articulation s'apprête à poser son talon 27 au sol, dans une situation où sa cuisse et sa jambe sont en extension c'est-à-dire sensiblement alignées, la palette 14 est écartée, et la jambe est libre de tourner par rapport à la cuisse non représentée. L'appui sur le sol du talon 27 engendre alors un effort de réaction du sol R1 situé au niveau du talon, qui rabat alors la palette 14 contre le corps 4, ce qui provoque le blocage de l'articulation, c'est-à-dire son accouplement.

Le porteur peut alors prendre fermement appui sur son articulation pour continuer son pas, car la jambe 26 ne risque alors pas de se replier sous sa cuisse, grâce au fait que l'articulation est nécessairement bloquée durant cette phase.

Au fur et à mesure que son pas se déroule, la zone d'application des efforts de réaction du sol sur la chaussure 27 avance le long de cette chaussure jusqu'à atteindre une région située vers l'avant de la semelle.

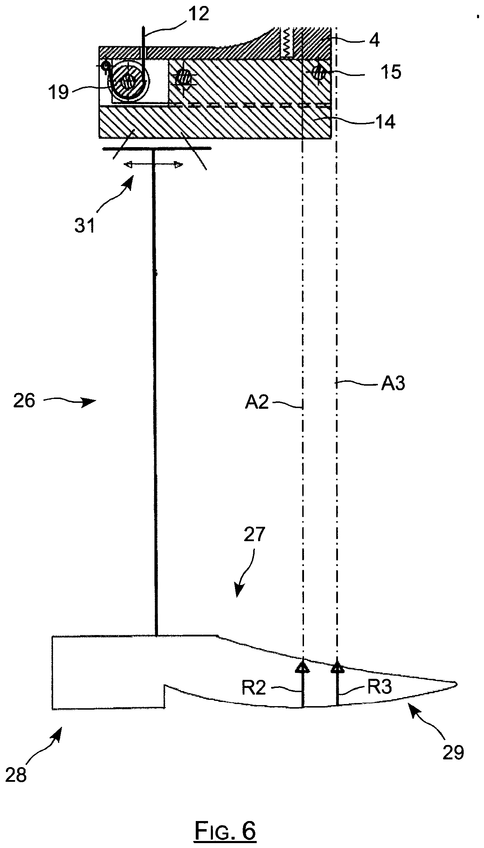

A ce stade, qui est illustré à la figure 6, l'axe d'application de la réaction R2 du sol passe d'une situation, représentée par A2, dans laquelle il est en arrière de l'axe 15, maintenant par là même la palette 14 en appui sur le corps 4, à une situation repérée par A3 dans laquelle la réaction R3 est décalée en avant de l'axe 15, provoquant de la sorte l'ouverture de la palette 14, et par là même la libération de l'articulation qui devient ainsi désaccouplée.

Comme illustré aux figures 5 et 6, la palette 14 comporte avantageusement des moyens pour ajuster la position de fixation de la jambe le long de cette palette 14, c'est-à-dire entre son extrémité avant et son extrémité arrière, ce qui permet d'ajuster le système au porteur de l'articulation pour affiner les instants de déclenchement du blocage et du déblocage de l'articulation durant la marche.

Concrètement l'embase supérieure 31 de fixation de la jambe artificielle à la palette 14 peut être fixée dans différentes régions le long de la face inférieure de la palette 14, celle-ci pouvant avantageusement être pourvue d'un rail de fixation orienté longitudinalement. Concrètement, le déblocage de l'articulation à la fin du pas a lieu d'autant plus tôt que la jambe 26 est située vers l'avant de la palette 14.

## Revendications

1. Mécanisme d'articulation d'une prothèse de jambe comprenant :
- un élément (1) destiné à être solidaire de la cuisse d'un sujet ;
- un autre élément (4, 14) destiné à être solidaire de la jambe (26) de la prothèse, cet autre élément comprenant un corps (4) et une palette basculante (14) articulée sur ce corps au niveau d'un axe (15) déporté vers l'avant selon le sens de la marche, cet autre élément (4, 14) étant destiné à être solidarisé à la jambe de la prothèse par la palette basculante (14) ;
- une bague intérieure (6) portée par l'un (1) des éléments et une bague extérieure (2) portée par l'autre élément (1) ;
- les bagues étant accouplées à rotation par l'intermédiaire d'un embrayage (7, 8, 8a) à coincement intercalé entre elles et pourvu d'un moyen de neutralisation (9, 10, 11) de l'accouplement des deux bagues comportant une roue (9) de commande;
- cette roue de commande étant accouplée par enroulement sans glissement, à au moins un lien (12) d'indexation de la roue dans l'une ou l'autre de deux positions de celle-ci par rapport à l'embrayage;
- une extrémité de ce lien (12) étant fixée à l'élément (4) solidarisé à la jambe par une fixation élastique (13) agissant sur ce lien (12) dans le sens de déblocage de l'embrayage ;
- l'autre extrémité de ce lien (12) étant fixée à la palette basculante (14) par l'intermédiaire d'un galet de renvoi (19) porté par le corps (4), de sorte que la fixation élastique du lien (12) tend à écarter la palette (14) du corps (4) et que le rabattement de la palette (14) contre le corps (4) agit sur le lien (12) à rencontre de son rappel élastique pour bloquer l'embrayage ;
- de telle manière qu'un appui sur le sol au niveau du talon (28) du pied (27) prolongeant la jambe (26) tend à rabattre la palette (14) pour bloquer l'articulation, et qu'un appui sur le sol au niveau de la pointe (29) du pied (27) tend à écarter la palette (14) pour débloquer l'articulation.

2. Mécanisme selon la revendication 1, comportant en outre des moyens pour ajuster la position de la jambe (26) sur la palette (14) à laquelle cette jambe (26) est fixée.

3. Mécanisme selon la revendication 1 ou 2, comprenant en outre un ressort de compression interposé entre la palette (14) et le corps (4) pour tendre continuellement à écarter la palette (14) du corps (4) et/ou amortir les chocs résultant de l'entrée en contact du talon avec le sol.

4. Mécanisme selon l'une des revendications 1 à 3, **caractérisé en ce que** l'embrayage à coincement comporte un corps de blocage formé par la bague intérieure (6) qui comporte au moins un logement (7) dont la surface en regard de la surface intérieure de la bague extérieure (2) formant un coin dans lequel, sous l'effet d'un ressort (8a), un rouleau (8) vient se loger et interdit toute rotation relative des bagues (2,6) dans le sens de la poussée du ressort (8a).

5. Mécanisme selon l'une des revendications 1 à 4, **caractérisé en ce que** le moyen de neutralisation de l'accouplement en rotation des deux bagues (2,6) comporte au moins la roue (9) susdite, laquelle porte, pour chaque rouleau (8), entre les deux bagues (2,6), un doigt (11) de limitation du déplacement des rouleaux (8) sous l'effet des ressorts (8a), mobile dans le logement (7) entre une position qui est dans le coin susdit pour empêcher le rouleau (8) de s'y loger et une position qui est au-delà dudit coin pour que le rouleau puisse s'y loger.

6. Mécanisme selon la revendication 5, caractérisé en ce le lien susdit est formé par un câble (12) qui s'enroule sans glissement sur une partie au moins de la périphérie de la roue (9), dans une gorge de celle-ci qui lui donne une fonction de poulie.

## Patentansprüche

1. Gelenkmechanismus einer Beinprothese mit:
- einem Element (1), das dazu bestimmt ist, mit dem Schenkel einer Person verbunden zu werden;
- einem weiteren Element (4, 14), das dazu bestimmt ist, mit dem Prothesenbein (26) verbunden zu werden, wobei dieses weitere Element einen Körper (4) und eine verschwenkbare Platte (14) aufweist, die im Bereich einer in Gehrichtung nach vorne versetzten Achse (15) gelenkig mit dem Körper verbunden ist und wobei dieses weitere Element (4, 14) dazu bestimmt ist, über die verschwenkbare Platte (14) mit dem Prothesenbein verbunden zu sein;
- einem inneren Ring (6), der durch eines (1) der Elemente gehalten ist, und einem äußeren Ring (2), der durch das andere Element (1) gehalten ist;
- wobei die Ringe mithilfe einer Klemmkupplung (7, 8, 8a) drehbar zusammengekuppelt sind, die zwischen diese eingebracht ist und die mit einem ein Steuerrad (9) aufweisenden Mittel (9, 10, 11) für ein Neutralisieren des Zusammenkuppelns der beiden Ringe versehen ist;
- wobei das Steuerrad durch einen schlupffreien Umlauf an ein Band (12) angekoppelt ist, um das Rad in die eine oder andere seiner beiden Stellungen bezüglich der Kupplung zu bringen;
- wobei ein Ende dieses Bandes (12) an dem mit dem Bein verbundenen Element (4) über eine elastische Befestigung (13) angebracht ist, die auf das Band (12) in Richtung einer Freigabe der Kupplung einwirkt;
- wobei das andere Ende dieses Bandes (12) an der verschwenkbaren Platte (14) mithilfe einer von dem Körper (4) gehaltenen Umlenkrolle (19) angebracht ist, so dass die elastische Befestigung des Bandes (12) dazu neigt, die Platte (14) vom Körper (4) zu entfernen und so dass die Annäherung der Platte (14) zum Körper (4) gegen den elastischen Zug des Bandes (12) wirkt, um die Kupplung zu blockieren;
- so dass auf diese Weise ein Abstützen der Ferse (28) des das Bein (26) verlängernden Fußes (27) auf dem Boden dazu neigt, die Platte (14) anzunähern, um das Gelenk zu blockieren, und so dass ein Abstützen im Bereich der Spitze (29) des Fußes (27) auf dem Boden dazu neigt, die Platte (14) zu entfernen, um das Gelenk freizugeben.

2. Mechanismus nach Anspruch 1, der ferner Mittel aufweist, um die Position des Beins (26) auf der Platte (14), an der dieses Bein (26) angebracht ist, einzustellen.

3. Mechanismus nach Anspruch 1 oder 2, der weiterhin eine Kompressionsfeder aufweist, die zwischen die Platte (14) und dem Körper (4) eingebracht ist, um die Platte (14) kontinuierlich eher vom Körper (4) zu entfernen und/oder die Stöße aufzunehmen, die durch das Auftreffen der Ferse auf dem Boden entstehen.

4. Mechanismus nach einem der Ansprüche 1 bis 3,
**dadurch gekennzeichnet, dass** die Klemmkupplung einen Blockierkörper aufweist, der von dem inneren Ring (6) gebildet ist, der wenigstens eine Aufnahme (7) aufweist, deren Oberfläche in Bezug auf die innere Oberfläche des äußeren Rings (2) eine Verengung bildet, in die eine Rolle (8) durch die Einwirkung einer Feder (8a) zu liegen kommt und jede relative Drehung der Ringe (2, 6) in Richtung der Federkraft (8a) unterbindet.

5. Mechanismus nach einem der Ansprüche 1 bis 4,
**dadurch gekennzeichnet, dass** das Mittel für ein Neutralisieren der Drehkupplung der beiden Ringe (2, 6) wenigstens das oben erwähnte Rad (9) aufweist, das für jede Rolle (8) zwischen den beiden Ringen (2, 6) einen Stift (11) aufweist, um die Verschiebung der Rollen (8) unter der Einwirkung der Federn (8a) zu begrenzen, der in der Aufnahme (7) zwischen einer Stellung, die in der oben erwähnten Verengung liegt, um zu verhindern, dass sich die Rolle (8) dort aufhält, und einer Stellung bewegbar ist, die abseits der Verengung liegt, so dass sich die Rolle dort aufhalten kann.

6. Mechanismus nach Anspruch 5, **dadurch gekennzeichnet, dass** das oben erwähnte Band von einem Strang (12) gebildet ist, der schlupffrei über wenigstens einen Teil des Umfangs des Rades (9) umläuft, in einer in diesem ausgebildeten Vertiefung, die diesem die Funktion einer Keilscheibe verleiht.

## Claims

1. A method for articulating a leg prosthesis, comprising:
- an element (1) intended to be secured to the thigh of a subject;
- another element (4, 14) intended to be secured to the leg (26) of the prosthesis, this other element comprising a body (4) and a tilting plate (14) articulated on this body at an axis (15) offset towards the front in the direction of walking, this other element (4, 14) being intended to be secured to the leg of the prosthesis by the tilting plate (14);
- an inner ring (6) carried by one (1) of the elements and an outer ring (2) carried by the other element (1);
- the rings being rotationally coupled by means of a wedging clutch (7, 8, 8a) interposed between them and provided with a means (9, 10, 11) for neutralising the coupling of the two rings and comprising a control wheel (9) :
- this control wheel being coupled, by slip-free winding, to at least one link (12) for locating the wheel in one or other of two positions thereof with respect to the clutch;
- one end of this link (12) being fixed to the element (4) secured to the leg by an elastic fixing (13) acting on this link (12) in the direction of release of the clutch;
- the other end of this link (12) being fixed to the tilting plate (14) by means of a return roller (19) carried by the body (4), so that the elastic fixing of the link (12) tends to separate the plate (14) from the body (4) and so that the folding of the plate (14) against the body (4) acts on the link (12) counter to its elastic return in order to lock the clutch;
- so that a pressing on the ground at the heel (28) of the foot (27) extending the leg (26) tends to fold the plate (14) in order to lock the articulation, and so that a pressing on the ground at the tip (29) of the foot (27) tends to move the plate (14) away in order to unlock the articulation.

2. A mechanism according to claim 1, further comprising means for adjusting the position of the leg (26) on the plate (14) to which this leg (26) is fixed.

3. A mechanism according to claim 1 or claim 2, further comprising a compression spring interposed between the plate (14) and the body (4) in order to tend continuously to move the plate (14) away from the body (4) and/or to dampen the shocks resulting from the heel coming into contact with the ground.

4. A mechanism according to one of claims 1 to 3, **characterised in that** the wedging clutch comprises a locking body formed by the inner ring (6), which comprises at least one housing (7), the surface of which opposite the internal surface of the outer ring (2) forming a corner in which, under the effect of a spring (8a), a roller (8) comes to be housed and prevents any relative rotation of the rings (2, 6) in the direction of the thrust of the spring (8a).

5. A mechanism according to one of claims 1 to 4, **characterised in that** the means for neutralising the rotational coupling of the two rings (2, 6) comprises at least the aforementioned wheel (9), which carries, for each roller (8), between the two rings (2, 6), a finger (11) for limiting the movement of the rollers (8) under the effect of the springs (8a) able to move in the housing (7) between a position that is in the aforementioned corner in to prevent the roller (8) from being held therein and a position that is beyond said corner so that the roller can be housed therein.

6. A mechanism according to claim 5, **characterised in that** the aforementioned link is formed by a cable (12) that winds without slip on at least part of the periphery of the wheel (9), in a groove therein that gives it a pulley function.
